# EUROPEAN PATENT APPLICATION

(11) **EP 3 573 071 A1**
(43) Date of publication of application: **27.11.2019**
(21) Application number: 18461558.1
(22) Date of filing: 23.05.2018
(51) Int. Cl.: G16H 40/67, A41D 13/12, A61B 5/0402, A61B 5/00

(54) **AN ELECTROCARDIOGRAPHY TELEMETRY SYSTEM AND METHOD**

(71) Applicant: Zieba, Bogumil, 90-437 Lodz (PL)
(72) Inventor: Zieba, Bogumil, 90-437 Lodz (PL)
(74) Representative: Kancelaria Eupatent.pl Sp. z.o.o

(57) **Abstract**

An electrocardiography telemetry system, comprising: an acquisition device, comprising: a shirt (10) comprising front electrodes (12) and back electrodes (13) for collecting ECG signals; a microcontroller (24) connected via signal path (23) to the electrodes (12, 13) for reading signals collected by the electrodes (12, 13) and converting the signals to digital ECG data; and a short-range communication device (21) configured to send the ECG data (24); a communication device (30) configured to read the ECG data transmitted by the short-range communication device (21) and to send the ECG data to a server (40); and wherein the server (40) is communicatively connected to a database (41) for storing the ECG data and comprises a SNET manager (44) configured to make the ECG data accessible at a social network (SNET) circle (124) in real time.

## Description

### TECHNICAL FIELD

The present invention relates to telemetry of cardiac activity, in particular to a electrocardiography telemetry.

### BACKGROUND

Telemetry systems can be implemented to acquire and transmit data from a remote source. Some telemetry systems provide information about user activities. Medical telemetry systems may comprise an alarm adapted to identify high risk patients and/or patients requiring special assistance.

The popularity and growth of social network sites and services has increased dramatically over the last few years. Present social network sites include Facebook(R), Google+(R), Twitter(R), MySpace(R), YouTube(R), Linkedln(R), Flicker(R) and the like. Such social networking (SNET) sites are typically web-based and organized around user profiles and/or collections of content accessible by members of the network. Membership in such social networks is comprised of individuals, or groupings of individuals, who are generally represented by profile pages and permitted to interact as determined by the social networking service. In many popular social networks, especially profile-focused social networks, activity centers on web pages or social spaces that enable members to view profiles, communicate and share activities, interests, opinions, status updates, audio/video content, etc., across networks of contacts. Social networking services might also allow members to track certain activities of other members of the social network, collaborate, locate and connect with existing friends, former acquaintances and colleagues, and establish new connections with other members. Individual members typically connect to social networking services through existing web-based platforms via a computing device, tablet or smartphone. Members often share a common bond, social status, or geographic or cultural connection with their respective contacts. Smartphone and games-based mobile social networking services are examples of rapidly developing areas.

A US patent application US20130283256 discloses a monitoring device and associated methods of use in communication with a telemetry system, which sends firmware updates from a telemetry system to a monitoring device. One or more sensors are coupled to a monitoring device that has a unique user ID. The sensors acquire user information selected from of at least one of, a user's activities, behaviors and habit information.

There is a need to further improve medical telemetry systems.

### SUMMARY

There is presented a electrocardiography telemetry system, comprising: an acquisition device, comprising: a shirt comprising front electrodes and back electrodes for collecting ECG signals; a microcontroller connected via signal path to the electrodes for reading signals collected by the electrodes and converting the signals to digital ECG data; and a short-range communication device configured to send the ECG data; a communication device configured to read the ECG data transmitted by the short-range communication device and to send the ECG data to a server; wherein the server is communicatively connected to a database for storing the ECG data and comprises a SNET manager configured to make the ECG data accessible at a social network (SNET) circle in real time.

Preferably, the communication device further comprises a post-processor to process the ECG data by denoising, equalizing and/or filtering.

Preferably, the server further comprises a post-processor to process the ECG data by denoising, equalizing and/or filtering.

Preferably, the communication device further comprises a data presentation module to present the ECG data via a graphical user interface.

Preferably, the server further comprises data analyzer configured to analyze the ECG data, detect abnormalities and to send a warning signal to the communication device or to external entities.

There is also presented a method for electrocardiography telemetry, comprising the steps of: acquiring ECG data of a user via an acquisition device, comprising: a shirt comprising front electrodes and back electrodes for collecting ECG signals; a microcontroller connected via signal path to the electrodes for reading signals collected by the electrodes and converting the signals to digital ECG data; and a short-range communication device configured to send the ECG data; reading the ECG data transmitted by the short-range communication device in a communication device configured send the ECG data to a server; and using the server, storing the ECG data in a database and making the data accessible at a social network (SNET) circle.

### BRIEF DESCRIPTION OF DRAWINGS

The system and method presented herein are shown by means of exemplary embodiments shown in a drawing, wherein:
Figs. 1A, 1B present a telemetry shirt;
Fig. 2 presents a general schematic of the system;
Fig. 3 presents a schematic of the acquisition module;
Fig. 4 presents a more detailed schematic of the system;
Fig. 5 presents an SNET circle;
Fig. 6 illustrates an embodiment of a social group 150.

### DETAILED DESCRIPTION

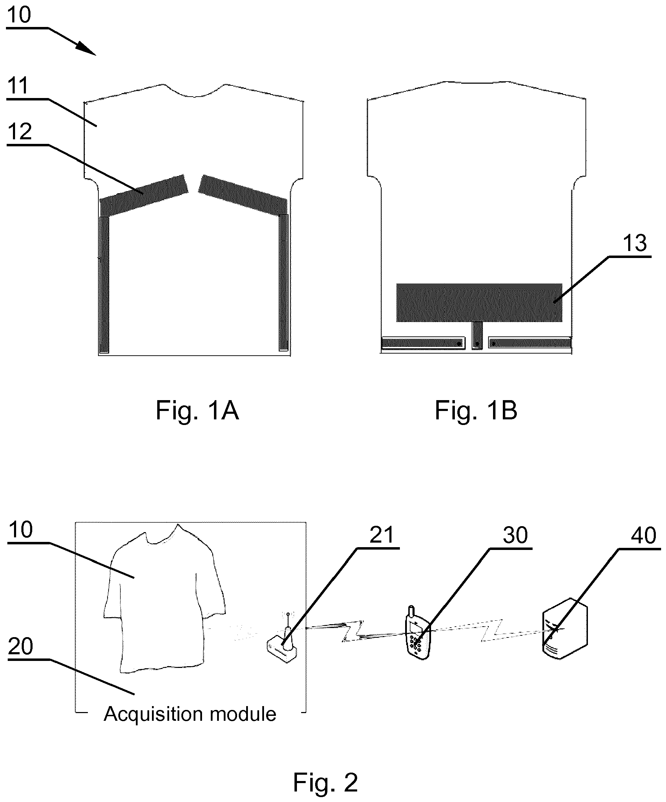

Figs. 1A, 1B present a telemetry shirt (as viewed from the front and back, respectively), such as a T-shirt, to be used with the telemetry system presented herein. The shirt may have a plurality of sizes (such as common sizes XS, S, M, L, XL etc) and shall be selected to closely fit the body of a particular user. The shirt 10 comprises base fabric 11 in which there are embedded front electrodes 12 and back electrodes 13 for collecting ECG (Electrocardiography) signals. The electrodes 12, 13 may be have a form of additional pieces of conductive fabric that are attached (e.g. sewn or glued) to the base fabric 11. Alternatively, the electrodes 12, 13 may be woven in the base fabric 11 as portions of the fabric woven with conductive thread.

Fig. 2 presents a schematic of th e system. The telemetry shirt 10 and a short-range communication module 21 form an acquisition module 20, which communicates with a communication device 30 such as a smartphone of the user. The communication device 30 reads the data sent by the short-range communication module and sends the ECG data to a server 40. The communication between the communication device 30 and the server 40 is handled by wireless means, such as, WiFi, GSM etc.

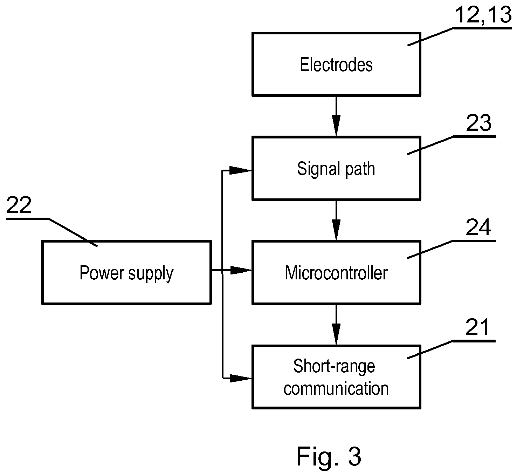

Fig. 3 presents a schematic of the acquisition module 20. The electrodes 12 and 13 are connected via a signal path 23 (e.g. a wire or a conductive thread integrated with the shirt 10, which may include additional powered amplification circuits) to a microcontroller 24. The microcontroller 24 is configured to convert the electrodes signal into digital data, for example by sampling signals of individual electrodes and optionally providing additional signal processing, such as de-noising, equalizing, amplifying etc. The digital signal from the microcontroller is transmitted by a short-range communication module 21, preferably low-power module, such as Bluetooth or module. The modules 21, 23, 24 are powered by a power supply 22, embedded with the shirt 10 or a connectable external supply, such as a battery or a power generator, such as a solar cell. The power supply shall preferably enable enough power to supply the acquisition module for at least 24 hours, enabling to perform a full-day ECG monitoring test, such as a test equivalent to one performed by standard Holter monitors.

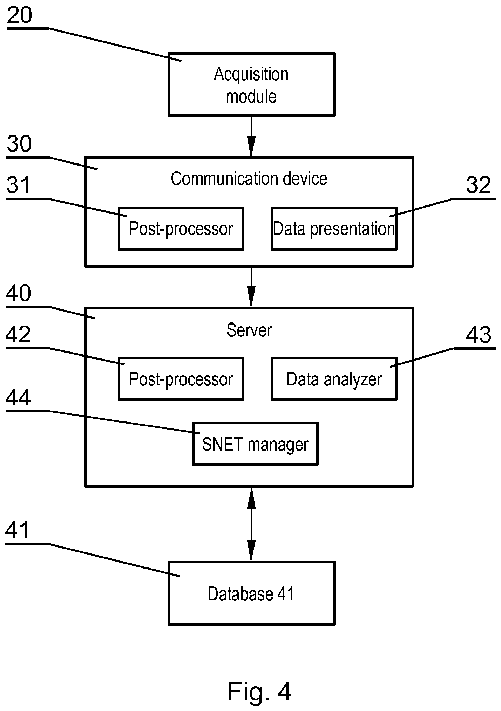

Fig. 4 presents a more detailed schematic of the system. The communication device 30 may comprise a post-processor module 31 for processing the received ECG data and further processing, such as denoising, equalizing and/or filtering. The communication device 30 may further comprise a presentation module 32 for presenting the measurement results, for example by presenting the ECG plot and/or alert signals indicative of detected ECG abnormalities. The presentation module 32 may obtain data from the post-processor 31 of the communication device or from the post-processor of the server 40. The server 40, to which the communication device 30 sends telemetry ECG data, is coupled to a database 41. Each acquisition module 20 is assigned its own unique identification, ID or asset tag or more fully explained hereafter. The data transmitted by the acquisition module 20, obtained from electrodes 12, 13 and its ID may be coded by appending a seed to digital data bits. In case the data from electrodes 12, 13 is only sampled by the data acquisition module 20 and not processed, further, the server 40 may perform the further processing by a post-processor 42, such as denoising, equalizing and/or filtering. The acquired data is stored in the database 41 along with the ID of the acquisition module. The server may further comprise a data analyzer 43 configured to analyze the collected ECG data and detect any abnormalities. In case an abnormality is detected, e.g. an abnormality related to a life threat, a warning signal is sent back to the communication device of the user and/or (preferably predefined) external entities, such as a communication device of a family member of the user and/or a doctor and/or a medical facility. The server further comprises a SNET manager 44 configured to present data from the database via the social network, e.g. to publish data on user social network account. The SNET manager is configured to publish data to a dedicated application utilizing SNET API (Application Program Interface) and allows to present the ECG plot in a graphical manner.

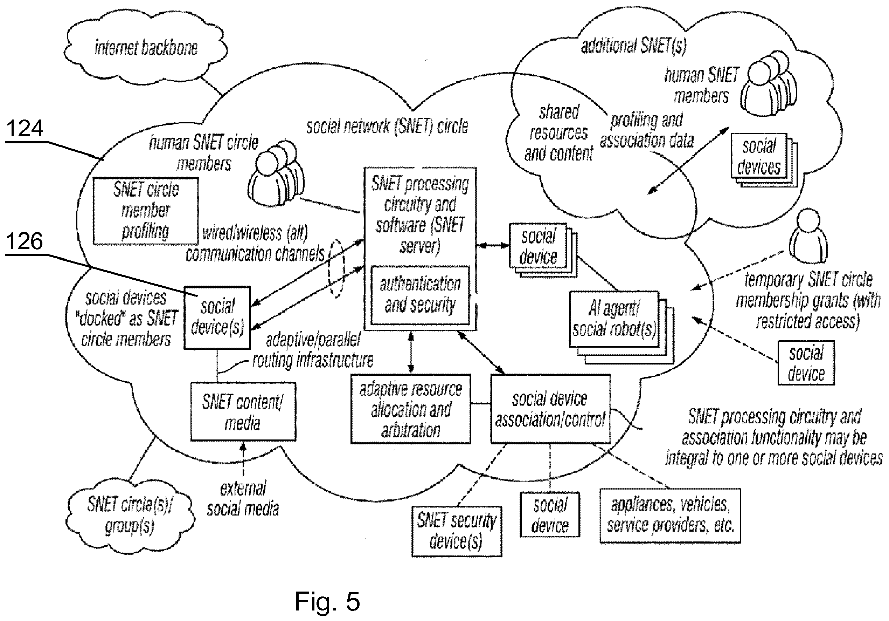

Fig. 5 presents a social network circle/group 124 ("SNET circle") comprising social devices 126, including the data acquisition device 20. The data acquisition device 20 can communicate with the SNET circle to send acquired data, via a network, such as Internet, or through the server 40, via the server 40 using the SNET manager 44. The SNET manager is configured to utilize a dedicated application of the social media service to publish the ECG data. Briefly, membership in the SNET circle 124 may comprise docked and undocked social devices 124 and human SNET circle members, as well as proxies thereof. Further, SNET circle 124 nodes may include device services and software (e.g., applications) of various types participating as members. By way of example, SNET circle members might include artificial intelligence agents/social robots, SNET security device(s), appliances, vehicles and service providers, common or authorized members/functionality of other SNET circles, and the like. Further, access to specific content and resources of a SNET circle may be shared with members of additional SNET(s), including remote or web-based applications. Such access can be conditioned on acceptable profiling and association data. Similarly, social devices or individuals may be granted temporary or ad hoc memberships, with or without restricted access. In the illustrated embodiment, formation, maintenance and operation of SNET circle is performed by standalone or distributed SNET processing circuitry and software. It is noted that the "SNET processing circuitry" may comprise hardware, software, applications, or various combinations thereof, and be configurable to support various functionalities disclosed herein. Further, the SNET processing circuitry may be included in a standalone server, server farm, cloud-based resources, Network System, system and/or the various types of devices described below, and incorporate authentication and security functionality. In addition, specialized middleware may also be utilized by SNETs according to the invention, including standardized middleware with an associated certification process. Interactions and interdependencies within the SNET circle may involve one or more of a social device association/control module, a SNET circle member profiling module, and an adaptive resource allocation and arbitration module. Distribution of internal and external SNET content/media can be accomplished in a variety of ways. For example, media distribution may involve an adaptive or parallel Network System routing infrastructure involving a wide variety of communication protocols and wired and/or wireless communications channels. SNET content/media may comprise, for example, various user-driven (advertising) channels, pictures, videos, links, online text, etc. Access to such content, as well as communications with and remote access to social devices of the SNET circle, may occur over an Network Systems backbone, cellular communication system, WAN, LAN, and the like.

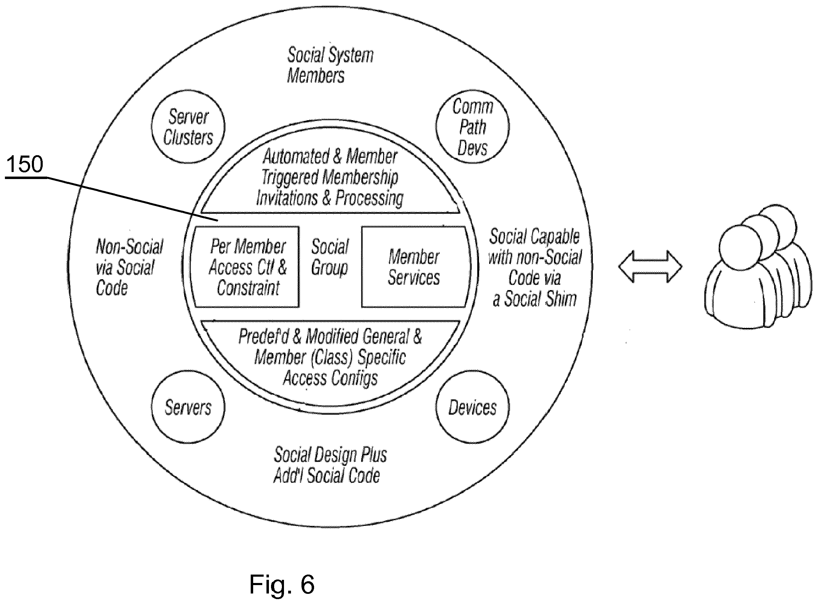

Fig. 6 illustrates an embodiment of a social group 150 comprising a variety of members that can communicate through their communication devices 30 and other devices, including but not limited to mobile devices. In this embodiment, membership in the social group may include a variety of novel social system members functioning in various capacities within the social group 150. As will be understood, certain of the social system members may support direct or indirect associations between the social group and human members/non-members and users. In the illustrated embodiment, social system members (or nodes) include one or more local or remote servers and server clusters that provide a support infrastructure for social group functionality and member operations (routing, data storage, services, etc.). Communications within the social group and with non-members may occur via dedicated or multifunction communication path devices. Social system members further include devices configured to operate as nodes within the social group. Social functionality in such devices and other social system members can be implemented through various means. For example, a device may have integral hardware/firmware/software to support social group access and member operations. Alternatively, a general purpose device a may include social code that enables participation in the social group. In a further embodiment, a device designed to include social functionality may participate in the social group through a combination of non-social code and a social shim layer or driver wrapper. In yet another embodiment, a member device having a social design may utilize additional social code, including code specific to a social group. Participation in the social group is supported through functionality that includes automated and member-triggered membership invitations and processing (membership management). More particularly, membership management may function to invite prospective members to participate in the social group through automatic, automated and member-triggered processes. For example, membership management might be configured by a human user to establish a social group by automatically inviting/accepting social system members having certain characteristics (such as devices owned or controlled by the user or acquaintances of the user). Processing of accepted invitations and unsolicited requests to join the social group may be conditioned upon input or authorization from an existing social system member(s) or human user(s) (e.g., through a user interface). Similarly, membership management may be configured to generate automated suggestions regarding which prospective members receive an invitation. Various other approaches, such as those described herein, can be used to establish membership in accordance with the invention. Access to and visibility of resources of a social group, including services and data, may be managed through general and member class-specific access configurations. For example, if membership in the social group includes family members and associated devices, a uniform access configuration (or separate device and human configurations) could be applied across the class in an automatic or automated manner. In other embodiments, access control and constraints are imposed on a per-member basis. The social group may offer a wide variety of member services, including both internal and external services accessible by social system members.

As one of the services, the social network may allow sharing of ECG data acquired by the acquisition device of one user to other users. The data is shared real-time, e.g. as soon as data is acquired by the acquisition device 20, it is sent to the server 40, which via the SNET manager publishes the data at the social network site of that particular user. The data is then instantaneously shared with other members of the network. Therefore, the ECG data can be shared with friends, family members and professionals (e.g. doctors or other members of medical staff) to monitor the health condition of the user. Moreover, the user can access their own data quickly, either by the communication device 30 via which the data is shared to the SNET or by communication another device which can access the SNET. The ECG data are presented as a graphical plot of the ECG signal.

Due to the fact that a new system for electrocardiography telemetry is presented herein, which applies hardware devices, the machine or transformation test is fulfilled and the idea is not abstract.

It can be easily recognized, by one skilled in the art, that the aforementioned method and system may be performed and/or controlled by one or more computer programs. Such computer programs are typically executed by utilizing the computing resources in a computing device. Applications are stored on a non-transitory medium. An example of a non-transitory medium is a nonvolatile memory, for example a flash memory or volatile memory, for example RAM. The computer instructions are executed by a processor. These memories are exemplary recording media for storing computer programs comprising computer-executable instructions performing all the steps of the computer-implemented method according the technical concept presented herein.

While the invention presented herein has been depicted, described, and has been defined with reference to particular preferred embodiments, such references and examples of implementation in the foregoing specification do not imply any limitation on the invention. It will, however, be evident that various modifications and changes may be made thereto without departing from the broader scope of the technical concept. The presented preferred embodiments are exemplary only, and are not exhaustive of the scope of the technical concept presented herein.

Accordingly, the scope of protection is not limited to the preferred embodiments described in the specification, but is only limited by the claims that follow.

## Claims

1. An electrocardiography telemetry system, comprising:
- an acquisition device, comprising:
- a shirt (10) comprising front electrodes (12) and back electrodes (13) for collecting ECG signals;
- a microcontroller (24) connected via signal path (23) to the electrodes (12, 13) for reading signals collected by the electrodes (12, 13) and converting the signals to digital ECG data; and
- a short-range communication device (21) configured to send the ECG data (24);
- a communication device (30) configured to read the ECG data transmitted by the short-range communication device (21) and to send the ECG data to a server (40); and
- wherein the server (40) is communicatively connected to a database (41) for storing the ECG data and comprises a SNET manager (44) configured to make the ECG data accessible at a social network (SNET) circle (124) in real time.

2. The system according to claim 1, wherein the communication device (30) further comprises a post-processor (31) to process the ECG data by denoising, equalizing and/or filtering.

3. The system according to claim 1, wherein the server (40) further comprises a post-processor (41) to process the ECG data by denoising, equalizing and/or filtering.

4. The system according to claim 1, wherein the communication device (30) further comprises a data presentation module (32) to present the ECG data via a graphical user interface.

5. The system according to claim 1, wherein the server (40) further comprises data analyzer (43) configured to analyze the ECG data, detect abnormalities and to send a warning signal to the communication device (30) or to external entities.

6. A method for electrocardiography telemetry, comprising the steps of:
- acquiring ECG data of a user via an acquisition device, comprising:
- a shirt (10) comprising front electrodes (12) and back electrodes (13) for collecting ECG signals;
- a microcontroller (24) connected via signal path (23) to the electrodes (12, 13) for reading signals collected by the electrodes (12, 13) and converting the signals to digital ECG data; and
- a short-range communication device (21) configured to send the ECG data (24);
- reading the ECG data transmitted by the short-range communication device (21) in a communication device (30) configured send the ECG data to a server (40); and
- using the server (40), storing the ECG data in a database (41) and making the data accessible at a social network (SNET) circle (124).
